# DEMANDE DE BREVET EUROPEEN

(11) **EP 3 125 142 A1**
(43) Date de publication de la demande: **01.02.2017**
(21) Numéro de dépôt: 16181476.9
(22) Date de dépôt: 27.07.2016
(51) Int. Cl.: G06F 19/00

(54) **PROCEDES ET DISPOSITIFS DE GESTION DE LA TRACABILITE DU STOCK D'UN CABINET DENTAIRE**

(30) Priorité: 27.07.2015 FR 1557137
(71) Demandeur: Exotec Dentaire, 21800 Sennecey les Dijon (FR)
(72) Inventeur: BLOIS, Eric, 21800 Crimolois (FR); ROSSI, Olivier, 21800 Crimolois (FR)
(74) Mandataire: Gevers SA

(57) **Abrégé**

Système de gestion de la traçabilité de produits dentaires au sein d'un cabinet dentaire comportant au moins un fauteuil de soin, qui comporte, géré par un dispositif de traitement de l'information au sein d'une base de données informatique, un ensemble de données représentant l'ensemble des produits dentaires, un ensemble de données l'ensemble des consultations effectuées par le cabinet contenant pour chaque consultation au moins la date et l'heure de la consultation ; l'identification du patient ; l'identification du type de consultation ; ledit système étant remarquable en ce qu'il comporte en outre un module de gestion de la traçabilité apte à générer un ensemble de données de traçabilité comportant pour chaque produit dentaire utilisé ; et un module de consultation de la traçabilité.

## Description

La présente invention concerne le domaine des procédés et dispositifs de gestion de la traçabilité du stock d'un cabinet dentaire.

Dans le cadre de la matériovigilance, la législation en vigueur impose au chirurgien dentiste de tenir un cahier de traçabilité des consommables, des dispositifs médicaux et des cycles de stérilisation utilisés dans son cabinet. Son objectif est de permettre d'identifier rapidement les patients exposés aux risques pour lesquels les produits d'un lot ou d'une série ont été utilisés et d'en surveiller les conséquences ; ou de pouvoir mettre en place les mesures adéquates de réduction ou d'élimination du risque par le retrait du produit, en retrouvant rapidement les lots qui auraient pu nuire aux patients. Cet objectif impose une rigueur en tout point du circuit.

Force est de constater que dans la plupart des cabinets dentaire, le domaine de la traçabilité des produits et de la stérilisation est souvent source d'inquiétude et de stress supplémentaire pour les praticiens déjà confrontés à une réglementation croissante. De plus, sa bonne gestion représente à elle seule une charge de travail supplémentaire non négligeable.

Pour permettre la traçabilité de l'usage des différents consommables et des produits utilisés lors de chaque consultation, les solutions existantes imposent une saisie de tous ces consommables et de tous ces produits utilisés lors de la consultation. Ce grand nombre de saisies est générateur de perte de temps, de stress et de risque d'erreur humaine.

La présente invention a pour but de résoudre les inconvénients précités en proposant une solution basées sur une gestion intelligente des sorties du stock. La solution proposée permet de gérer quels sont les consommables et les produits en service à un moment donné et pour un fauteuil de soin donné. Une solution technique permet alors de reconstruire l'information des consommables et des produits utilisés lors d'une consultation précise à partir du type de consultation, de son heure et de la connaissance des consommables et produits en circulation à ce moment et au niveau du fauteuil de soin utilisé.

Ainsi, l'objectif de traçabilité est atteint sans nécessiter la saisie systématique de tous les produits et consommables utilisés lors de chaque consultation. Cette solution diminue notablement les saisies nécessaires et donc le temps passé en saisie et le risque d'erreur lors de ces saisies.

L'invention concerne un système de gestion de la traçabilité de produits dentaires au sein d'un cabinet dentaire comportant au moins un fauteuil de soin, qui comporte, géré par un dispositif de traitement de l'information au sein d'une base de données informatique :
- un ensemble de données représentant l'ensemble des produits dentaires gérés par le cabinet appelé stock ;
- un ensemble de données représentant l'ensemble des consultations effectuées par le cabinet contenant pour chaque consultation au moins :
   ∘ la date et l'heure de la consultation ;
   ∘ l'identification du patient ;
   ∘ l'identification du type de consultation ;
ledit système est remarquable en ce qu'il comporte en outre :
- un module de gestion de la traçabilité apte à générer un ensemble de données de traçabilité comportant pour chaque produit dentaire utilisé :
   ∘ la date et l'heure de la mise en circulation du produit au sein du cabinet ;
   ∘ le numéro de lot ou de cycle de stérilisation du produit dentaire ;
   ∘ la date et l'heure de fin de circulation ;
- un module de consultation de la traçabilité.

Ledit module de gestion de la traçabilité des produits dentaires comporte :
- des moyens d'enregistrement du début de la mise en circulation d'un produit dentaire au sein de l'ensemble de données de traçabilité, pour l'enregistrement d'au moins :
   ∘ la date et l'heure de la mise en circulation ;
   ∘ le numéro de lot ou de cycle de stérilisation du produit dentaire ;
- des moyens d'enregistrement de la fin de circulation du produit dentaire au sein de l'ensemble de données de traçabilité, pour l'enregistrement au moins de la date et de l'heure de fin de circulation du produit.

Le cabinet dentaire comportant une pluralité de fauteuils de soin, le module de gestion de la traçabilité des produits dentaires comporte en outre des moyens d'enregistrement du fauteuil de soin au niveau duquel le produit dentaire a été mis en circulation.

Par ailleurs, le module de gestion de la traçabilité des produits dentaires comporte en outre des moyens de vérification lors de l'enregistrement du début de la mise en circulation, qu'un même produit n'est pas déjà en circulation pour le même fauteuil de soin avec un numéro de lot ou de cycle de stérilisation différent.

Ledit module de consultation de la traçabilité des produits dentaires comporte des moyens de croisement sur la base temporelle de la base de consultation et de l'ensemble de données de traçabilité pour déterminer les produits utilisés lors d'une consultation.

Par ailleurs, le système comporte par le module de gestion de la traçabilité :
- une étape d'enregistrement du début de la mise en circulation d'un produit dentaire, au sein de l'ensemble de données de traçabilité, comportant l'enregistrement d'au moins :
   ∘ la date et l'heure de la mise en circulation ;
   ∘ le numéro de lot ou de cycle de stérilisation du produit dentaire ;
- une étape d'enregistrement de la fin de circulation du produit dentaire, au sein de l'ensemble de données de traçabilité, comportant au moins l'enregistrement de la date et de l'heure de fin de circulation du produit.

Le cabinet comportant une pluralité de fauteuils de soin, l'étape d'enregistrement du début de la mise en circulation comporte en outre l'enregistrement du fauteuil de soin concerné par la circulation.

Par ailleurs, il comporte en outre une étape de vérification lors de l'enregistrement du début de la mise en circulation, qu'un même produit n'est pas déjà en circulation pour le même fauteuil de soin avec un numéro de lot ou de cycle de stérilisation différent.

Un autre objet de l'invention concerne un procédé de consultation de données de traçabilité au sein d'un système selon l'invention remarquable en ce qu'il comporte par le module de consultation de la traçabilité :
- une étape d'identification du produit et du numéro de lot ou de cycle de stérilisation ;
- une étape d'extraction des informations de circulation associées comprenant la plage horaire de circulation et le fauteuil de soin associé ;
- une étape de parcours de la base de consultation à la recherche des consultations ayant eu lieu dans la plage horaire concernée et au niveau du fauteuil de soin associé ; et
- une étape de filtrage des consultations ayant nécessité l'utilisation du type de produit dont le lot ou le cycle de stérilisation est recherché.

Par ailleurs, le procédé comporte par le module de consultation de la traçabilité :
- une étape d'identification d'une consultation recherchée ;
- une étape d'extraction des informations associées à la consultation contenant la date et l'heure de la consultation et les types de produits dentaires utilisés ;
- une étape de parcours de la base de données de traçabilité à la recherche des numéros de lots et de cycles de stérilisation des produits en circulation au niveau du fauteuil associé ; et
- une étape d'identification identifie parmi ces numéros de lots et de cycles de stérilisation des produits correspondant aux types de produits utilisés lors de la consultation.

Un autre objet de l'invention concerne un programme d'ordinateur comprenant des instructions adaptées à la mise en oeuvre de chacune des étapes du procédé selon l'invention lorsque ledit programme est exécuté sur un ordinateur.

Un dernier objet de l'invention concerne un moyen de stockage d'informations, amovible ou non, partiellement ou totalement lisible par un ordinateur ou un microprocesseur comportant des instructions de code d'un programme d'ordinateur pour l'exécution de chacune des étapes du procédé selon l'invention.

Dans un mode particulier de réalisation, des étapes du procédé précité sont déterminées par des instructions de programmes d'ordinateurs.

En conséquence, l'invention vise aussi un programme d'ordinateur sur un support d'informations, ce programme étant susceptible d'être mis en oeuvre par un microprocesseur, ce programme comprenant des instructions adaptées à la mise en oeuvre des étapes du procédé tel que mentionné ci-dessus.

Ce programme peut utiliser n'importe quel langage de programmation, et être sous la forme de code source, code objet, ou de code intermédiaire entre code source et code objet, tel que dans une forme partiellement compilée, ou dans n'importe quelle autre forme souhaitable.

L'invention vise aussi un support d'informations lisible par un microprocesseur, et comprenant des instructions d'un programme d'ordinateur tel que mentionné ci-dessus.

Le support d'informations peut être n'importe quelle entité ou dispositif capable de stocker le programme. Par exemple, le support peut comprendre un moyen de stockage, tel qu'une ROM, par exemple une ROM de microcircuit, ou encore un moyen d'enregistrement magnétique, par exemple un disque dur, ou encore une mémoire flash.

D'autre part, le support d'informations peut être un support transmissible tel qu'un signal électrique ou optique, qui peut être acheminé via un câble électrique ou optique, par radio ou par d'autres moyens. Le programme selon l'invention peut être en particulier téléchargé sur une plateforme de stockage d'un réseau de type Internet.

Alternativement, le support d'informations peut être un circuit intégré dans lequel le programme est incorporé, le circuit étant adapté pour exécuter ou pour être utilisé dans l'exécution du procédé en question.

Le support d'informations et le programme d'ordinateur précités présentent des caractéristiques et avantages analogues au procédé qu'ils mettent en oeuvre.

D'autres particularités et avantages de l'invention apparaîtront encore dans la description ci-après en relation avec les dessins annexés, donnés à titre d'exemples non limitatifs :
- la figure 1 est une représentation schématique de l'architecture du système de gestion du stock et de la traçabilité selon l'invention ;
- la figure 2 est une représentation schématique de l'architecture du sous système de gestion de la traçabilité conforme à l'invention ;
- la figure 3 est un ordinogramme des différentes étapes du procédé utilisé par le module de gestion de la traçabilité suivant l'invention ;
- la figure 4 est une représentation schématique de l'architecture du sous système de gestion de la traçabilité selon l'invention ;
- la figure 5 est un ordinogramme des différentes étapes du procédé de gestion de la traçabilité suivant l'invention ;
- la figure 6 est un ordinogramme illustrant les différentes étapes de l'exploitation des données à partir d'un produit ;
- la figure 7 un ordinogramme illustrant les différentes étapes de l'exploitation des données à partir d'une consultation ;
- la figure 8 est un bloc-diagramme schématique d'un dispositif de traitement de l'information pour la mise en oeuvre du procédé suivant l'invention.

Dans le cadre de ce document, un produit correspond à toute fourniture dentaire soumise ou non aux réglementations relative à la matériovigilance ou non. Les consommables sont des produits destinés à être utilisés dans le cadre des consultations sans réutilisation possible. Ces consommables peuvent être utilisés en une seule fois lors d'une seule consultation ou être partitionables comme par exemple les amalgames où les anesthésiants qui sont consommés au fur et à mesure sur plusieurs consultations. Les articles de stérilisation sont des produits pouvant être réutilisés mais devant subir un cycle de stérilisation entre deux utilisations.

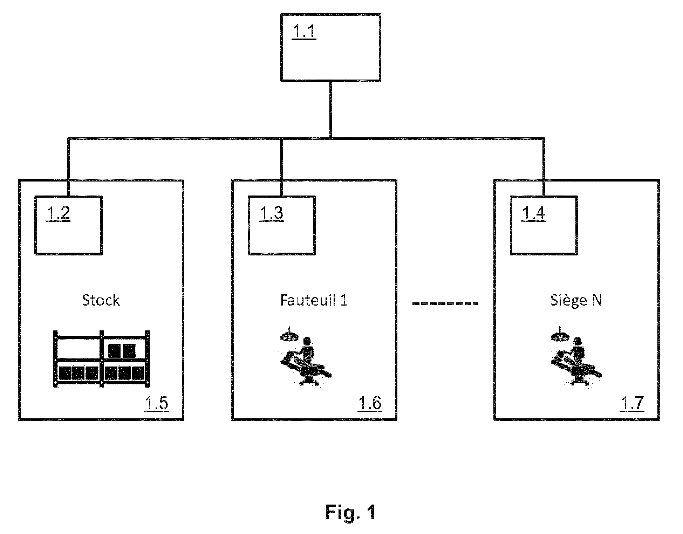

La **figure 1** illustre un exemple d'architecture du système de gestion du stock et de la traçabilité selon l'invention. Cet exemple considère la présence d'un stock **1.5** de produits dentaires au sein d'un cabinet dentaire. Le cabinet dispose de un ou plusieurs fauteuils de soin permettant à un ou plusieurs praticiens de pratiquer des consultations. Ces fauteuils **1.6** et **1.7** sont des postes de travail supposés autonomes et disposant de leur propre matériel.

Le système de gestion du stock et de la traçabilité est composé d'un applicatif logiciel exécuté sur un dispositif de traitement de l'information **1.1.** Ce dispositif de traitement de l'information peut être un simple ordinateur personnel, un serveur ou encore tout autre dispositif programmable permettant l'exécution des programmes de gestion décrits plus loin. Ce dispositif peut être localisé au sein du cabinet dentaire ou à distance chez un prestataire correspondant à un fonctionnement en ligne ou dans le nuage (*cloud* en anglais) selon la terminologie courante.

Dans l'exemple de la **figure 1****,** des terminaux de consultation/saisie **1.2, 1.3** et **1.4** sont disponibles au sein du cabinet dans les lieux concernés par la gestion des stocks et de la traçabilité. En premier lieu un terminal de consultation/saisie **1.2** est installé au niveau du stock **1.5** du cabinet en produits dentaires. Ensuite, un terminal de consultation/saisie **1.3, 1.4** est également installé au niveau de chaque fauteuil de soin dans le cabinet.

Chaque terminal de consultation/saisie est connecté à l'applicatif logiciel de gestion du stock et de la traçabilité. Cette connexion peut se faire via un réseau local filaire ou sans fil lorsque l'applicatif logiciel est hébergé sur un serveur local dans le cabinet. La connexion peut également être faite via un réseau de communication de données, typiquement le réseau Internet, si l'applicatif logiciel de gestion du stock est hébergé sur un serveur distant. Dans ce dernier cas, les communications sont avantageusement chiffrées pour en préserver la confidentialité. Par exemple un tunnel chiffré ou VPN (*Virtual Private Network* en anglais) peut être établi entre les terminaux de consultation/saisie et l'applicatif logiciel de gestion.

Avantageusement les produits dentaires sont identifiés à l'aide d'étiquettes représentant des codes, par exemple du type code à deux dimension ou « Datamatrix » tel que normalisé par la norme ISO/CEI 16022. Les terminaux de consultation/saisie sont alors avantageusement équipés d'un dispositif de lecture de codes Datamatrix appelés couramment « douchette ». Bien évidemment tout autre type de code et d'étiquette peut permettre l'identification des produits.

Le dispositif de consultation/saisie peut être constitué de tout type de dispositif de traitement de l'information. Il s'agit typiquement d'un ordinateur pouvant prendre tout facteur de forme, ordinateur de bureau, ordinateur portable, tablette, téléphone intelligent (*smartphone* en anglais) ou autre.

Le lecteur comprendra que cette architecture n'est qu'un exemple type qui peut subir des variations en fonction de la taille du cabinet sans influence sur l'invention. Le système entier, applicatif logiciel de gestion et terminaux de consultation/saisie peuvent être hébergés sur un seul dispositif de traitement de l'information, par exemple un ordinateur portable utilisé pour toutes les opérations. A l'inverse, l'architecture peut s'étendre à une ferme (*cluster* en anglais) de serveurs, éventuellement virtuels, hébergés dans le nuage.

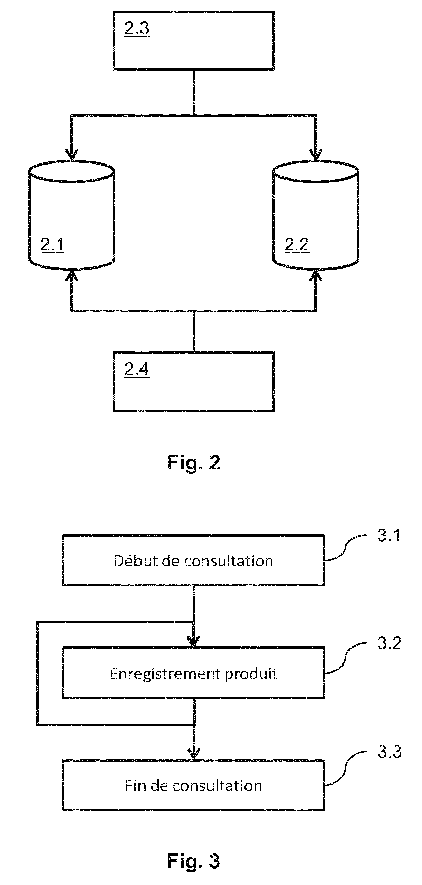

La **figure 2** illustre l'architecture du sous système de gestion de la traçabilité dans le cadre d'une gestion classique.

Un premier ensemble de données **2.1** représente le stock, c'est-à-dire l'ensemble des produits dentaires gérés par le cabinet. Les modules de pure gestion du stock ne sont pas décrits ici, car ils sont connus et ne participent pas directement à l'invention. Le stock est géré sous la forme d'un ensemble de produits. Chaque produit se voit attribuer des attributs tels que un code produit identifiant la nature du produit, un numéro de lot identifiant le produit au sein des autres produits de même nature. Des informations complémentaires peuvent être présents en fonction du type de produit, par exemple une date de péremption, le nom du produit, la référence du fournisseur etc. Le produit de stérilisation se voient attribuer des informations spécifiques au cycle de stérilisation, typiquement une date de stérilisation, un numéro de cycle de stérilisation, l'identification de l'opérateur ayant assurer la stérilisation, l'identification de l'appareil de stérilisation utilisé etc. Bien évidemment, la gestion du stock implique la présence d'une date d'entrée dans le stock et d'une date de sortie du stock.

Un second ensemble de données **2.2** représente la base de consultation, c'est-à-dire l'ensemble des consultations effectuées par le cabinet. Cette base de consultation est typiquement gérée par un logiciel de suivi de patientèle spécifique. Dans certains modes de réalisation, la base de consultation est également gérée par le même logiciel qui gère le stock. Chaque consultation est associée à un patient et à un type de soin effectué. Elle est également associée à un lieu, le fauteuil de soin utilisé, sauf éventuellement dans le cas d'un cabinet ne possédant qu'un seul fauteuil de soin. Elle est encore associée à une datation comprenant typiquement la date et l'heure de la consultation. Elle peut également être associée au praticien ayant effectué la consultation, typiquement dans le cas où plusieurs praticiens exercent dans le cabinet. Cette liste n'est évidemment pas exhaustive.

La **figure 2** est une illustration logique du sous-système et ne prétend pas illustrer l'architecture matérielle hébergeant ces données. En particuliers ces ensembles de données peuvent être hébergées par une ou plusieurs bases de données informatiques. Ces bases de données peuvent être implémentées au sein du dispositif de traitement de l'information hébergeant l'applicatif logiciel de gestion de stock et de la traçabilité où sur un dispositif séparé mais connecté de manière à ce que l'applicatif puisse consulter et modifier ces bases de données.

Le module de gestion de la traçabilité **2.3** est le module qui permet d'enregistrer les informations de traçabilité. Ce module est typiquement un module de l'applicatif logiciel de gestion du stock et de la traçabilité **1.1** et peut être piloté depuis l'un des terminaux de saisie. C'est le module qui permet d'enregistrer lors de chaque consultation les produits dentaires utilisés. Ce module permet d'associer à chaque consultation de la base de consultation **2.2** les produits dentaires du stock **2.1** qui ont été utilisés.

Le module de consultation de traçabilité **2.4** permet quant à lui la consultation à postériori des informations de traçabilité. Ce module est également typiquement un module de l'applicatif logiciel de gestion du stock et de la traçabilité **1.1** et peut être piloté depuis l'un des terminaux de saisie. C'est le module qui permet de retrouver à partir d'un produit donné, les consultations au cours desquelles il a été utilisé. Il permet également à l'inverse de retrouver tous les produits utilisés pour une consultation donnée. Avantageusement, il permet également de retrouver la liste des produits utilisés lors des différentes consultations d'un patient donné. Ces consultations se font en exploitant les liens établis entre la base des consultations **2.2** et le stock **2.1.**

La **figure 3** illustre le procédé utilisé par le module de gestion de la traçabilité **2.3** selon un exemple de réalisation.

Lors d'une étape **3.1,** l'enregistrement des informations de traçabilité liées à la consultation commence. Lors d'une étape **3.2,** l'utilisation d'un premier produit utilisé lors de cette consultation est enregistrée. Ceci ce fait typiquement par l'utilisation de la douchette pour lire l'étiquette imprimée sur le produit. Les informations lues sur cette étiquette permettent d'identifier le produit dans le stock et de le lier à la consultation. Cette étape se répète pour tous les produits utilisés lors de la consultation. Lors de l'étape **3.3,** l'enregistrement se termine pour la consultation courante.

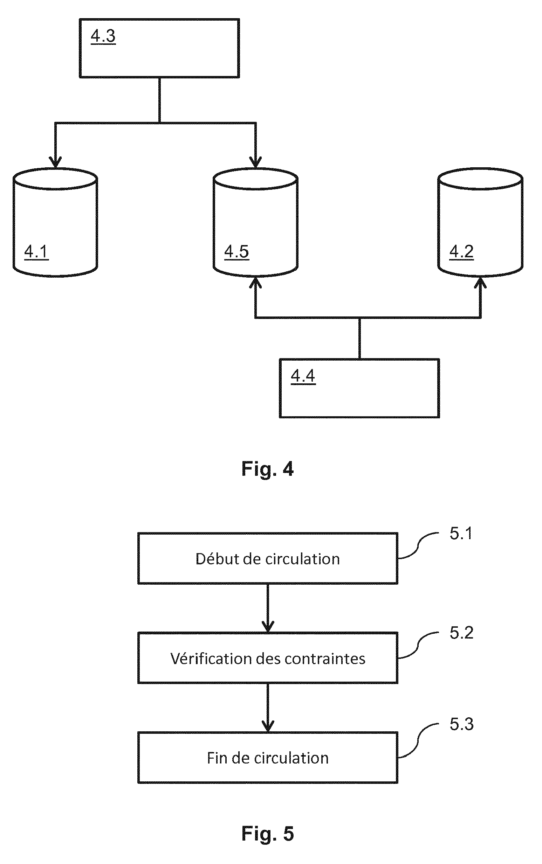

La **figure 4** illustre l'architecture du sous système de gestion de la traçabilité selon un exemple de réalisation de l'invention. On parle dans cet exemple de chrono-traçabilité.

On retrouve dans cet exemple de traçabilité un ensemble de données **4.1** représentant le stock de manière similaire à l'ensemble **2.1.** De même, l'ensemble de données **4.2** représente la base de consultation de manière similaire à l'ensemble de données **2.2.** L'ensemble **4.5** est par contre nouveau et représente les données de traçabilité générées par le module de traçabilité **4.2.**

Le module de traçabilité **4.3** au contraire du module **2.3** ne s'intéresse pas aux consultations de la base de consultation. Ce module se contente d'enregistrer la circulation des produits. Par circulation, on entend l'opération par laquelle un produit quitte le stock du cabinet pour être mis en service au niveau d'un fauteuil de soin jusqu'au moment où il n'est plus disponible. Il peut ne plus être disponible car épuisé au niveau du fauteuil de soin ou parce qu'il nécessite un nouveau cycle de stérilisation avant de pouvoir être réutilisé. La circulation d'un produit se caractérise donc par une mise en circulation qui est l'opération de mise à disposition du produit au niveau d'un fauteuil de soin et par une fin de circulation qui est l'opération signalant la fin de sa disponibilité au niveau du fauteuil.

Ainsi, une saisie lors de la mise en circulation et une autre lors de la fin de circulation suffisent à générer les données de traçabilité. Il n'est plus nécessaire d'effectuer une saisie lors de chaque utilisation du produit dans une consultation.

Le module de consultation de traçabilité **4.4** permet la consultation des informations de traçabilité. Ce module n'a pas besoin d'accéder aux donner du stock. Il permet la consultation des informations de traçabilité sur la base d'un croisement des données de traçabilité et des données de consultations. Comme il n'y a pas de lien explicite entre ces données, le croisement se fait sur la base des informations temporelles. Chaque consultation contient le type de la consultation. Sur la base du type de consultation on peut identifier les différents types de produits dentaires utilisés pour la consultation. Il suffit alors de chercher dans les données de traçabilité, pour chaque type de produit nécessaire, le lot en circulation à l'heure de la consultation au niveau du fauteuil de soin utilisé pour déterminer précisément les produits utilisés lors de cette consultation.

Ce procédé de croisement des données de consultation et des données de traçabilité sur la base temporelle nécessite pour être fiable le respect des contraintes suivantes :
- Il ne peut y avoir qu'un seul lot par produit en circulation en même temps pour un même fauteuil de soin.
- Il ne peut y avoir qu'un seul numéro de cycle de stérilisation en circulation en même temps pour un même fauteuil de soin.

Ces contraintes sont normalement respectées dans le fonctionnement normal d'un cabinet dentaire. Mais avantageusement, le module de gestion de la traçabilité 4.3 comprend un module de vérification de contrainte permettant d'interdire la mise en circulation de deux lots ou de deux numéros de cycle de stérilisation pour un même produit.

La **figure 5** illustre le procédé de gestion de la traçabilité **4.3** selon un exemple de réalisation de l'invention.

Lors d'une première étape **5.1,** le module de gestion de la traçabilité enregistre le début de la circulation au sein de l'ensemble de données de traçabilité. Sont alors mémorisé dans les données de traçabilité le numéro de lot du produit, la date et l'heure de mise en circulation et, dans le cas où le cabinet possède plusieurs fauteuils de soin, le fauteuil où le lot est mis en circulation. S'il s'agit d'un article de stérilisation, c'est le numéro de cycle de stérilisation qui est mémorisé au lieu du numéro de lot.

Lors d'une étape optionnelle **5.2,** les contraintes sont vérifiées. Plus particulièrement il s'agit de vérifier qu'un même produit n'est pas déjà en circulation pour le même fauteuil avec un numéro de lot différent ou un numéro de cycle de stérilisation différent.

Lors de l'étape **5.3,** lorsque le produit a été utilisé et n'est plus disponible pour le fauteuil, le module de traçabilité enregistre la date et l'heure de fin de circulation du produit associé à son numéro de lot ou de cycle de stérilisation au sein de l'ensemble de données de traçabilité.

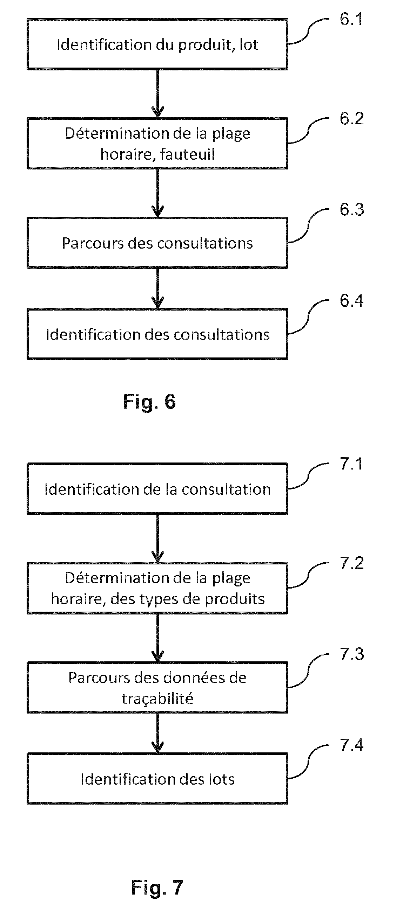

La **figure 6** illustre l'exploitation des données à partir d'un produit selon un exemple de réalisation de l'invention. Dans cet exemple, on illustre la consultation des données de traçabilité à partir d'un produit identifié par son numéro de lot ou de cycle de stérilisation pour retrouver les consultations et donc les patients ayant été traités par ce produit.

Lors d'une première étape **6.1,** le module de consultation de la traçabilité identifie le produit et le numéro de lot, ou de cycle de stérilisation dans la base des données de traçabilité.

Lors d'une étape **6.2,** les informations de circulation du lot ou du cycle de stérilisation associées au produit sont extraites. Ces informations contiennent la plage horaire de circulation et le fauteuil de soin associé.

Lors d'une étape **6.3,** le module de consultation de la traçabilité parcours la base de consultation à la recherche des consultations ayant eu lieu dans la plage horaire concernée et au niveau du fauteuil de soin associé.

Lors de l'étape **6.4,** le module de consultation de la traçabilité identifie les consultations ayant utilisé le produit dentaire recherché. Cette identification consiste en un filtre des consultations ayant nécessité l'utilisation du type de produit dont le lot ou le cycle de stérilisation est recherché.

Ainsi, on peut déterminer en toute fiabilité les patients ayant été traités par un lot particulier, ou cycle particulier, d'un produit dentaire donné. Ce qui est l'un des buts de la traçabilité.

La **figure 7** illustre l'exploitation des données à partir d'une consultation selon un exemple de réalisation de l'invention.

Lors d'une première étape **7.1,** le module de consultation de la traçabilité identifie la consultation recherchée. Cette identification peut, par exemple, être faite à partir du nom du patient.

Lors d'une étape **7.2,** les informations associées à la consultation sont extraites. Ces informations contiennent la date et l'heure de la consultation et les types de produits dentaires utilisés.

Lors d'une étape **7.3,** le module de consultation de la traçabilité parcours la base de données de traçabilité à la recherche des numéros de lots et de cycles de stérilisation des produits en circulation au niveau du fauteuil associé.

Lors de l'étape **7.4,** le module de consultation de la traçabilité identifie parmi ces numéros de lots et de cycles de stérilisation, les produits correspondant aux types de produits utilisés lors de la consultation.

Ainsi, on peut déterminer en toute fiabilité les lots de produits, ou leur cycle de stérilisation, ayant été utilisé lors d'une consultation particulière. Ce qui est un autre des buts de la traçabilité.

Avantageusement un système de gestion de la traçabilité selon l'invention peut proposer le mode de gestion de la traçabilité tel qu'illustré par la **figure 2** et le mode de chrono traçabilité tel qu'illustré par la **figure 3****,** le praticien pouvant sélectionner le mode de gestion de la traçabilité qu'il souhaite mettre en oeuvre dans son cabinet.

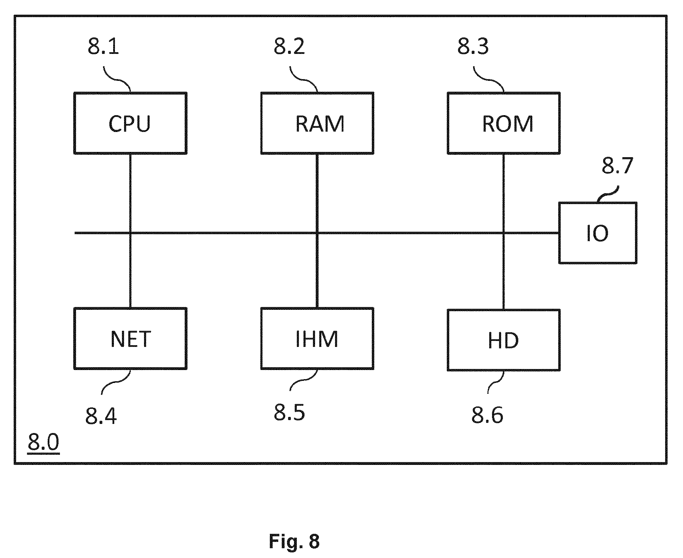

La **figure 8** est un bloc-diagramme schématique d'un dispositif de traitement de l'information **8.0** pour la mise en oeuvre d'un ou plusieurs modes de réalisation de l'invention. Le dispositif **8.0** de traitement de l'information peut être un périphérique tel qu'un micro-ordinateur, un poste de travail ou d'un terminal mobile de télécommunication. Le dispositif **8.0** comporte un bus de communication connecté à:
- une unité centrale de traitement **8.1,** tel qu'un microprocesseur, notée CPU ;
- une mémoire à accès aléatoire **8.2,** notée RAM, pour mémoriser le code exécutable du procédé de réalisation de l'invention ainsi que les registres adaptés à enregistrer des variables et des paramètres nécessaires pour la mise en oeuvre du procédé selon des modes de réalisation de l'invention, la capacité de mémoire de celui-ci peut être complété par une mémoire RAM optionnelle connectée à un port d'extension, par exemple ;
- une mémoire morte **8.3,** notée ROM, pour stocker des programmes informatiques pour la mise en oeuvre des modes de réalisation de l'invention ;
- une interface réseau **8.4** est normalement connectée à un réseau de communication sur lequel des données numériques à traiter sont transmis ou reçus. L'interface réseau **8.4** peut être une seule interface réseau, ou composée d'un ensemble d'interfaces réseau différentes (par exemple filaire et sans fil, interfaces ou différents types d'interfaces filaires ou sans fil). Des paquets de données sont envoyés sur l'interface réseau pour la transmission ou sont lues à partir de l'interface de réseau pour la réception sous le contrôle de l'application logiciel exécuté dans le processeur **8.1 ;**
- une interface utilisateur **8.5** pour recevoir des entrées d'un utilisateur ou pour afficher des informations à un utilisateur;
- un support de stockage optionnel **8.6** noté HD ;
- un module d'entrée/sortie **8.7** pour la réception / l'envoi de données depuis / vers des périphériques externes tels que disque dur, support de stockage amovible ou autres.

Le code exécutable peut être stocké dans une mémoire morte **8.3,** sur le support de stockage **8.6** ou sur un support amovible numérique tel que par exemple un disque. Selon une variante, le code exécutable des programmes peuvent être reçu au moyen d'un réseau de communication, via l'interface réseau **8.4,** afin d'être stocké dans l'un des moyens de stockage du dispositif de communication **8.0,** tel que le support de stockage **8.6,** avant d'être exécuté.

L'unité centrale de traitement **8.1** est adaptée pour commander et diriger l'exécution des instructions ou des portions de code logiciel du programme ou des programmes selon l'un des modes de réalisation de l'invention, instructions qui sont stockées dans l'un des moyens de stockage précités. Après la mise sous tension, le CPU **8.1** est capable d'exécuter des instructions stockées dans la mémoire RAM principale **8.2,** relatives à une application logicielle, après que ces instructions aient été chargées de la ROM par exemple. Un tel logiciel, lorsqu'il est exécuté par le processeur **8.1,** provoque les étapes des organigrammes présentés en illustration de l'invention pour être exécutées.

Dans ce mode de réalisation, l'appareil est un appareil programmable qui utilise un logiciel pour mettre en oeuvre l'invention. Toutefois, à titre subsidiaire, la présente invention peut être mise en oeuvre dans le matériel (par exemple, sous la forme d'un circuit intégré spécifique ou ASIC).

Naturellement, pour satisfaire des besoins spécifiques, une personne compétente dans le domaine de l'invention pourra appliquer des modifications dans la description précédente.

Bien que la présente invention ait été décrite ci-dessus en référence à des modes de réalisation spécifiques, la présente invention n'est pas limitée aux modes de réalisation spécifiques, et les modifications qui se trouvent dans le champ d'application de la présente invention seront évidentes pour une personne versée dans l'art.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Système de gestion de la traçabilité de produits dentaires au sein d'un cabinet dentaire comportant au moins un fauteuil de soin, qui comporte, géré par un dispositif de traitement de l'information au sein d'une base de données informatique :
- un ensemble de données représentant l'ensemble des produits dentaires gérés par le cabinet appelé stock ;
- un ensemble de données représentant l'ensemble des consultations effectuées par le cabinet contenant pour chaque consultation au moins :
∘ la date et l'heure de la consultation ;
∘ l'identification du patient ;
∘ l'identification du type de consultation ;
**caractérisé en ce qu'**il comporte en outre :
- un module de gestion de la traçabilité apte à générer un ensemble de données de traçabilité comportant pour chaque produit dentaire utilisé :
∘ la date et l'heure de la mise en circulation du produit au sein du cabinet ;
∘ le numéro de lot ou de cycle de stérilisation du produit dentaire ;
∘ la date et l'heure de fin de circulation ;
- un module de consultation de la traçabilité.

2. Système selon la revendication 1, **caractérisé en ce que** le module de gestion de la traçabilité des produits dentaires comporte :
- des moyens d'enregistrement du début de la mise en circulation d'un produit dentaire au sein de l'ensemble de données de traçabilité, pour l'enregistrement d'au moins :
∘ la date et l'heure de la mise en circulation ;
∘ le numéro de lot ou de cycle de stérilisation du produit dentaire ;
- des moyens d'enregistrement de la fin de circulation du produit dentaire au sein de l'ensemble de données de traçabilité, pour l'enregistrement au moins de la date et de l'heure de fin de circulation du produit.

3. Système selon la revendication 2, **caractérisé en ce que**, le cabinet dentaire comportant une pluralité de fauteuils de soin, le module de gestion de la traçabilité des produits dentaires comporte en outre des moyens d'enregistrement du fauteuil de soin au niveau duquel le produit dentaire a été mis en circulation.

4. Système selon l'une des revendication 1 à 3, **caractérisé en ce que** le module de gestion de la traçabilité des produits dentaires comporte en outre des moyens de vérification lors de l'enregistrement du début de la mise en circulation, qu'un même produit n'est pas déjà en circulation pour le même fauteuil de soin avec un numéro de lot ou de cycle de stérilisation différent.

5. Système selon la revendication 1, **caractérisé en ce que** le module de consultation de la traçabilité des produits dentaires comporte des moyens de croisement sur la base temporelle de la base de consultation et de l'ensemble de données de traçabilité pour déterminer les produits utilisés lors d'une consultation.

6. Procédé de gestion de la traçabilité des produits dentaires au sein d'un système selon la revendication 1, **caractérisé en ce qu'**il comporte par le module de gestion de la traçabilité :
- une étape d'enregistrement du début de la mise en circulation d'un produit dentaire, au sein de l'ensemble de données de traçabilité, comportant l'enregistrement d'au moins :
∘ la date et l'heure de la mise en circulation ;
∘ le numéro de lot ou de cycle de stérilisation du produit dentaire ;
- une étape d'enregistrement de la fin de circulation du produit dentaire, au sein de l'ensemble de données de traçabilité, comportant au moins l'enregistrement de la date et de l'heure de fin de circulation du produit.

7. Procédé selon la revendication 6, **caractérisé en ce que** le cabinet comportant une pluralité de fauteuils de soin, l'étape d'enregistrement du début de la mise en circulation comporte en outre l'enregistrement du fauteuil de soin concerné par la circulation.

8. Procédé selon l'une des revendications 6 ou 7, **caractérisé en ce qu'**il comporte en outre :
- une étape de vérification lors de l'enregistrement du début de la mise en circulation, qu'un même produit n'est pas déjà en circulation pour le même fauteuil de soin avec un numéro de lot ou de cycle de stérilisation différent.

9. Procédé de consultation de données de traçabilité au sein d'un système selon la revendication 1, **caractérisé en ce qu'**il comporte par le module de consultation de la traçabilité :
- une étape d'identification du produit et du numéro de lot ou de cycle de stérilisation ;
- une étape d'extraction des informations de circulation associées comprenant la plage horaire de circulation et le fauteuil de soin associé ;
- une étape de parcours de la base de consultation à la recherche des consultations ayant eu lieu dans la plage horaire concernée et au niveau du fauteuil de soin associé ; et
- une étape de filtrage des consultations ayant nécessité l'utilisation du type de produit dont le lot ou le cycle de stérilisation est recherché.

10. Procédé de consultation de données de traçabilité au sein d'un système selon la revendication 1, **caractérisé en ce qu'**il comporte par le module de consultation de la traçabilité :
- une étape d'identification d'une consultation recherchée ;
- une étape d'extraction des informations associées à la consultation contenant la date et l'heure de la consultation et les types de produits dentaires utilisés ;
- une étape de parcours de la base de données de traçabilité à la recherche des numéros de lots et de cycles de stérilisation des produits en circulation au niveau du fauteuil associé ; et
- une étape d'identification identifie parmi ces numéros de lots et de cycles de stérilisation des produits correspondant aux types de produits utilisés lors de la consultation.

11. Programme d'ordinateur comprenant des instructions adaptées à la mise en oeuvre de chacune des étapes du procédé selon l'une quelconque des revendications 6 à 10 lorsque ledit programme est exécuté sur un ordinateur.

12. Moyen de stockage d'informations, amovible ou non, partiellement ou totalement lisible par un ordinateur ou un microprocesseur comportant des instructions de code d'un programme d'ordinateur pour l'exécution de chacune des étapes du procédé selon l'une quelconque des revendications 6 à 10.
